(19) Europäisches Patentamt
European Patent Office
Office européen des brevets

(11) Veröffentlichungsnummer: **0 287 927 B1**

(12) **EUROPÄISCHE PATENTSCHRIFT**

(45) Veröffentlichungstag der Patentschrift: **04.03.92**

(51) Int. Cl.5: **A61K 6/00**

(21) Anmeldenummer: **88105767.3**

(22) Anmeldetag: **12.04.88**

# (54) Konditionierflüssigkeiten von Zahn- oder Knochensubstanz.

(30) Priorität: **24.04.87 DE 3713667**

(43) Veröffentlichungstag der Anmeldung:
**26.10.88 Patentblatt 88/43**

(45) Bekanntmachung des Hinweises auf die Patenterteilung:
**04.03.92 Patentblatt 92/10**

(84) Benannte Vertragsstaaten:
**AT BE CH DE ES FR GB IT LI NL SE**

(56) Entgegenhaltungen:
**DE-A- 2 365 257**
**US-A- 4 064 138**

**N.T.I.S. TECH NOTES, September 1988, Seiten 829,1-2, NBS technology update, Springfield, Va, US; ""Ultrasimplified" procedure developed for bonding composites to teeth"**

(73) Patentinhaber: **BAYER AG**

**W-5090 Leverkusen 1 Bayerwerk(DE)**

(72) Erfinder: **Asmussen, Erik, Dr.**
**Brudevaenget 15**
**DK-3520 Farum(DK)**
Erfinder: **Munksgaard, Erik Chr., Dr.**
**Enebaerhaven 306**
**DK-2980 Kokkedal(DK)**

EP 0 287 927 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Flüssigkeiten zur Konditionierung von defekter Zahn- oder Knochensubstanz für eine Versorgung mit plastischem Kunststoffmaterial.

Besonders im Dentalbereich werden härtende plastische Kunststoffmaterialien als Füllungsmaterialien bei der Versorgung von Kavitäten an der Zahn- oder Knochensubstanz verwendet. Als härtende Kunststoffmaterialien werden im allgemeinen Füllungen auf Acrylatbasis bevorzugt. Diese polymeren Füllungen haben jedoch den Nachteil, daß sie schlecht auf dem Zahnbein oder dem Knochen haften bleiben. Um dieses Problem zu lösen hat man z.B. bisher teilweise Unterschneidungen vorgenommen; dazu war es erforderlich, über den angegriffenen Bereich hinaus, beachtliche Mengen an frischem Zahnbein zu entfernen.

Um diese Nachteile zu vermeiden, hat man das Zahnbein oder den Knochen auf verschiedene Weise vorbehandelt, um die Haftung des Kuststoffmaterials zu erhöhen.

So ist es bekannt, das Zahnbein oder die Schmelzoberfläche mit starken Säuren, z.B. Phosphorsaure, anzuätzen und dann die Füllung vorzunehmen (Zidan et al., Scand. J. Dental Res. 88, 348 bis 351 (1980)). Abgesehen von de Reizwirkung der starken Säure im Mundbereich, ist die Haftung der Füllung unzureichend.

Es ist außerdem bekannt, das Zahnbein mit Ethylendiamintetra essigsäure vorzubehandeln und dann mit einem Beschichtungsmittel aus einem aliphatischen Aldehyd oder einem Keton und einem olefinisch ungesättigten Monomer, z.B. einem Ester der Acryl- oder Methacrylsäure, zu versehen (EP-A 0 141 324 und EP-A 0 199 057).

Erfindungsgemäß wurden Flüssigkeiten zur Konditionierung von Zahn- oder Knochensubstanz gefunden, die in wäßriger Lösung eine Säure mit einem $pK_S$-Wert von kleiner als +5 und
eine amphotere Aminoverbindung mit einem $pK_S$-Wert im Bereich von 3,9 bis 12,5 und einem $pK_B$-Wert im Bereich von 10,5 bis 13,5,
in einem solchen Verhältnis zueinander, daß
die Lösung einen pH-Wert im Bereich von 0,1 bis 3,5 aufweist,
enthalten.

Die neuen erfindungsgemäßen Flüssigkeiten konditionieren die Zahn- oder Knochensubstanz vor einer Beschichtung mit einem Grundierungsmittel (Primer oder Liner). Auf der auf diese Weise vorbehandelten Zahn- oder Knochensubstanz tritt eine feste Bindung eines aufgetragenen plastischen Kunststoffmaterials ein.

Säuren für die erfindungsgemäßen Flüssigkeiten weisen eine Säurestärke [$pK_S$-Wert] von kleiner als 5, bevorzugt von -9 bis +5, auf. Die Löslichkeit der Säuren in Wasser ist im allgemeinen größer als 0,5 Gew.-%, bevorzugt größer als 1 Gew.-%.

Beispielsweise seien die folgenden Säuren genannt: Brenztraubensäure, Zitronensäure, Oxalsäure, Phosphorsäure und Salpetersäure.

Amphotere Aminoverbindungen für die erfindungsgemäßen Flüssigkeiten weisen eine Säurestärke [$pK_S$-Wert] im Bereich von 3,9 bis 12,5, bevorzugt von 9,0 bis 10,6, und eine Basenstärke [$pK_B$-Wert] im Bereich von 10,5 bis 13,5, bevorzugt von 11,5 bis 12,5 auf.

Vorzugsweise seien amphotere Aminoverbindungen der Formel

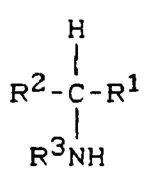

in der

- $R^1$ für eine Carboxylgruppe steht,
- $R^2$ Wasserstoff, gegebenenfalls durch Hydroxy, Thio, Methylthio, Carboxy, Carbonamid, Amino, Phenyl, Hydroxy-phenyl oder die Gruppen

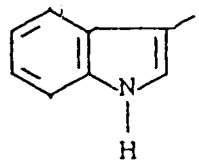 , 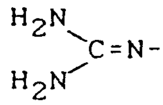 , 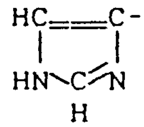

substituiertes $C_1$-$C_4$-Alkyl,

$R^3$ Wasserstoff oder Phenyl bedeutet oder

die Reste $R^2$ und $R^3$ durch einen Propylenrest verbunden sein können, oder

in der

$R^1$ für Wasserstoff steht,

$R^2$ die Gruppe

$$-A-NH_3X$$

in der

A für einen zweibindigen Alkylenrest mit 1 bis 6 Kohlenstoffatomen und

X für Halogen steht,

bedeutet und

$R^3$ Wasserstoff bedeutet,

genannt.

Beispielsweise seien die folgenden amphoteren Aminoverbindungen genannt: Glycin, Serin, Threonin, Cystein, Thyrosin, Asparagin, Glutamin, Alanin, Valin, Leucin, Isoleucin, Prolin, Methionin, Phenylalanin, Tryptophan, Lysin, Arginin, Histidin, N-Phenylglycin, Ethylendiaminhydrochlorid, Ethylendiaminhydrobromid, Propylendiaminhydrochlorid, Propylendiaminhydrobromid, Butylendiaminhydrochlorid and Butylendiaminhydrobromid.

Besonders bevorzugte amphotere Aminoverbindungen sind Glycin, Phenylalanin, Lysin und Ethylendiaminhydrochlorid.

Es wurde auch ein Verfahren zur Herstellung von Flüssigkeiten zur Konditionierung von Zahn- oder Knochensubstanz gefunden, das dadurch gekennzeichnet ist, daß man eine wäßrige Lösung einer Säure mit einem $pK_S$-Wert von kleiner als 5 und

eine amphotere Aminoverbindung mit einem $pK_S$-Wert im Bereich von 3,9 bis 12,5 und einen $pK_B$-Wert im Bereich von 10,5 bis 13,5,

umsetzt, wobei ein pH-Wert im Bereich von 0,1 bis 3,5 eingehalten wird.

Die Einhaltung des pH-Wertes für das erfindungsgemäße Verfahren kann in an sich bekannter Weise, z.B. mit Hilfe geeigneter Indikatoren oder mit Hilfe potentiometrischer Meßverfahren (Ullmann Band 5, 926 bis 936 (1980)) erfolgen.

Die Komponenten werden im allgemeinen bei der Herstellung der erfindungsgemäßen Flüssigkeiten unter kräftigem Rühren zusammengegeben. Die Zusammengabe der Komponenten erfolgt im allgemeinen bei Raumtemperatur, beispielsweise im Temperaturbereich von 0 bis 30 ° C.

Bei der Anwendung trägt man die erfindungsgemäßen Flüssigkeiten auf die defekte Zahn- oder Knochensubstanz, beispielsweise in eine Kavität, auf. Bevorzugt seien hier Kavitäten im Zahnschmelz (Enamel) oder Zahnbein (Dentin) genannt.

Nach dem Auftragen der erfindungsgemäßen Flüssigkeiten werden sie im allgemeinen, z.B. mit warmer Luft, getrocknet.

Vor der Versorgung der defekten Zahn- oder Knochensubstanz mit dem plastischen Kunststoffmaterial erfolgt nach der Konditionierung mit den erfindungsgemäßen Flüssigkeiten vorzugsweise eine Beschichtung mit einem Grundierungsmaterial.

Es seien hier besonders Grundierungsmaterialien genannt, wie sie in der EP-A 0 141 324 und der EP-A 0 199 057 beschrieben werden.

Besonders bevorzugt werden Grundierungsmaterialien, die einen Alkehyd oder ein Keton und ein ungesättigtes Monomer mit aktivem Wasserstoff enthalten.

Als Aldehyde seien hier Formaldehyd, Verbindungen die Formaldehyd freisetzen können, Acetaldehyd, Propionaldehyd, Butyraldehyd und Glutaraldehyd genannt. Insbesondere bevorzugt ist der Glutaraldehyd.

Als Ketone seien hier Cyclopentanon, Benzophenon, Cyclohexanon, 2,4-Pentandion und Kampferchinon genannt. Insbesondere bevorzugt wird Kampferchinon.

Als olefinisch ungesättigte Monomere mit aktivem Wasserstoff (Broensted-Säure) sei Acrylsäureester,

Methacrylsäureester und Acrylsäure- bzw. Methacrylsäureurethane mit OH-, $NH_2$-, NH-, SH- oder PH-Gruppen genannt. Insbesondere bevorzugt wird das Hydroxyethylmethacrylat.

Insbesondere bevorzugt sind Grundierungsmaterialien, die 1 bis 50 Gew.-% eines aliphatischen Aldehyds mit 1 bis 20 Kohlenstoffatomen und 5 bis 80 Gew.-% eines olefinisch ungesättigten Monomers mit wenigstens einem aktiven Wasserstoffatom in Form von OH-, $NH_2$- oder CH-Gruppen und gegebenenfalls Wasser und/oder ein toxikologisch akzeptables organisches Lösungsmittel enthalten. Die härtenden Kunststoffmaterialien werden im wesentlichen durch das Anwendungsgebiet bestimmt. So können beispielsweise im Dentalbereich für die Polymerisation nur Monomere eingesetzt werden, die physiologisch unbedenklich sind und die im Mundbereich polymerisieren können. Solche Monomeren für Zahnfüllungen sind an sich bekannt (E. Asmussen, Plastflyldningsmaterialer Of Boghandel, Kopenhagen 1981).

Als Kunststoffmaterialien seien beispielsweise Massen aus Acrylat- und/oder Methacrylatmonomeren, geeigneten Katalysatoren, Startern, Beschleunigern und Füllstoffen genannt.

Durch die Konditionierung der defekten Zahn- oder Knochensubstanz mit den erfindungsgemäßen Flüssigkeiten erhält man überraschenderweise eine Basis für die Versorgung mit Kunststoffmaterialien, die eine hohe Haltbarkeit und Festigkeit der Reparatur gewährleistet.

Beispiele 1 bis 35 (Herstellung)

Die erfindungsgemäßen Lösungen (Beispiele 1 bis 21) werden hergestellt, indem man entweder die Säure oder das amphotere Amin vorlegt und die andere Komponente unter starkem Rühren so zugibt, daß der erfindungsgemäße pH-Wert eingestellt ist.

In der folgenden Tabelle 1 sind in den Beispielen 1 bis 21 und 25 bis 35 erfindungsgemäße Flüssigkeiten und in den Beispielen 22 bis 24 Vergleichssubstanzen aufgeführt.

Tabelle 1

| Beispiel Nr. | Flüssigkeit zur Konditionierung | pH-Wert |
|---|---|---|
| 1 | 33,0 Gew.-% Phosphorsäure,<br>61,3 Gew.-% Wasser,<br>5,7 Gew.-% N-Phenyl-glycin | 0,2 |
| 2 | 2,4 Gew.-% Salpetersäure,<br>91,9 Gew.-% Wasser,<br>5,7 Gew.-% N-Phenyl-glycin | 1,0 |
| 3 | 9,6 Gew.-% Brenztraubensäure,<br>86,4 Gew.-% Wasser,<br>4,0 Gew.-% N-Phenyl-glycin | 1,5 |
| 4 | 18,0 Gew.-% Phosphorsäure,<br>76,1 Gew.-% Wasser<br>5,9 Gew.-% Glycin | 1,5 |
| 5 | 9,1 Gew.-% Brenztraubensäure,<br>81,8 Gew.-% Wasser<br>9,1 Gew.-% Lysin-hydrochlorid | 1,9 |

Tabelle 1 (Fortsetzung)

| Beispiel Nr. | Flüssigkeit zur Konditionierung | pH-Wert |
|---|---|---|
| 6 | 9,1 Gew.-% Brenztraubensäure,<br>81,8 Gew.-% Wasser<br>9,1 Gew.-% Glycin | 2,8 |
| 7 | 19,2 Gew.-% Zitronensäure,<br>66,0 Gew.-% Wasser<br>14,8 Gew.-% Glycin | 2,8 |
| 8 | 19,2 Gew.-% Zitronensäure,<br>69,9 Gew.-% Wasser<br>10,9 Gew.-% Glycin | 2,6 |
| 9 | 19,2 Gew.-% Zitronensäure,<br>74,6 Gew.-% Wasser<br>6,2 Gew.-% Glycin | 2,4 |
| 10 | 19,2 Gew.-% Zitronensäure,<br>77,1 Gew.-% Wasser<br>3,7 Gew.-% Glycin | 2,2 |
| 11 | 19,2 Gew.-% Zitronensäure,<br>79,0 Gew.-% Wasser<br>1,8 Gew.-% Glycin | 2,0 |
| 12 | 9,1 Gew.-% Brenztraubensäure,<br>81,8 Gew.-% Wasser<br>9,1 Gew.-% Glycin | 2,4 |
| 13 | 9,1 Gew.-% Brenztraubensäure,<br>81,8 Gew.-% Wasser<br>9,1 Gew.-% Ethylendiamin-hydrochlorid | |
| 14 | 8,4 Gew.-% Phosphorsäure,<br>80,0 Gew.-% Wasser<br>11,6 Gew.-% Glycin | 2,2 |
| 15 | 6 Gew.-% Essigsäure,<br>86 Gew.-% Wasser<br>8 Gew.-% Glycin | |

Tabelle 1 (Fortsetzung)

| Beispiel Nr. | Flüssigkeit zur Konditionierung | pH-Wert |
|---|---|---|
| 16 | 10 Gew.-% Weinsäure,<br>82 Gew.-% Wasser<br>8 Gew.-% Glycin | 2,8 |
| 17 | 10 Gew.-% Weinsäure,<br>85 Gew.-% Wasser<br>5 Gew.-% Glycin | 2,4 |
| 18 | 10 Gew.-% Weinsäure,<br>88 Gew.-% Wasser<br>2 Gew.-% Glycin | 2,2 |
| 19 | 10 Gew.-% Apfelsäure,<br>81 Gew.-% Wasser<br>9 Gew.-% Glycin | 2,8 |
| 20 | 10 Gew.-% Apfelsäure,<br>84 Gew.-% Wasser<br>6 Gew.-% Glycin | 2,4 |
| 21 | 10 Gew.-% Apfelsäure,<br>87 Gew.-% Wasser<br>3 Gew.-% Glycin | 2,0 |
| | Zum Vergleich | |
| 22 | 35 Gew.-% Phosphorsäure,<br>65 Gew.-% Wasser | 0,1 |
| 23 | 10 Gew.-% Brenztraubensäure<br>90 Gew.-% Wasser | 1,0 |
| 24 | 15 Gew.-% Ethylendiamin-tetraessigsäure | 7,4 |

Tabelle 1 (Fortsetzung)

| Beispiel Nr. | Flüssigkeit zur Konditionierung | pH-Wert |
|---|---|---|
| 25 | 19,2 Gew.-% Zitronensäure<br>79,0 Gew.-% Wasser<br>1,8 Gew.-% Glycin | 2,0 |
| 26 | 19,2 Gew.-% Zitronensäure<br>77,1 Gew.-% Wasser<br>3,7 Gew.-% Glycin | 2,2 |
| 27 | 19,2 Gew.-% Zitronensäure<br>74,6 Gew.-% Wasser<br>6,2 Gew.-% Glycin | 2,4 |
| 28 | 19,2 Gew.-% Zitronensäure<br>66,0 Gew.-% Wasser<br>14,8 Gew.-% Glycin | 2,8 |
| 29 | 9,6 Gew.-% Brenztraubensäure<br>86,0 Gew.-% Wasser<br>4,4 Gew.-% Glycin | 2,0 |
| 30 | 9,2 Gew.-% Brenztraubensäure<br>82,4 Gew.-% Wasser<br>8,4 Gew.-% Glycin | 2,4 |
| 31 | 8,5 Gew.-% Brenztraubensäure<br>76,1 Gew.-% Wasser<br>15,4 Gew.-% Glycin | 2,8 |
| 32 | 9,5 Gew.-% Maleinsäure<br>86,0 Gew.-% Wasser<br>4,5 Gew.-% Glycin | 2,0 |
| 33 | 9,4 Gew.-% Maleinsäure<br>84,5 Gew.-% Wasser<br>6,1 Gew.-% Glycin | 2,4 |
| 34 | 9,2 Gew.-% Maleinsäure<br>82,9 Gew.-% Wasser<br>7,9 Gew.-% Glycin | 2,8 |
| 35 | 10,0 Gew.-% Oxalsäure<br>78,0 Gew.-% Wasser<br>12,0 Gew.-% Glycin | 2,4 |

Beispiel 36 (Beschichtungsmittel)

Als Beschichtungsmittel wird eine Zubereitung aus
5 Gew.-% Glutaraldehyd,

35 Gew.-% Hydroxyethyl-methacrylat und
60 Gew.-% Wasser
verwendet.

Beispiel 37 (Prüfung der Bindungsstärke)

In dem Beispiel wird die Bindungstärke (Scherfestigkeit) zwischen Zahnbein (Dentin) bzw. Zahnschmelz (Enamel) und einer handelsüblichen Kunststoffüllmasse gemessen.

Für den Test werden herausgezogene und im feuchten Zustand aufbewahrte Menschenzähne benutzt. Die Zähne werden durch Guß in Epoxidharz eingelagert; durch Naßschleifen wird eine flache Oberfläche erzeugt. Das abschließende Schleifen erfolgt mit Carbonpapier 1000.

Beispiel Nr. 1 bis 6 und 22, 23, 24 wurde nach Bowen [J. Dent. Res. 1965, 44, 690 bis 695] gemessen.

Andere Beispiele wurden nach Munksgaard, Iric & Asmussen [J. Dent. Res. 64 (12), 1409 bis 1411 (1985)] gemessen.

Es wurde eine handelsübliche Kunststoffüllmasse eingesetzt.

Daraufhin wird die Oberfläche mit einer Flüssigkeit der Beispiele 1 bis 35 jeweils 60 Sekunden lang behandelt. Danach wird die behandelte Stelle mit destilliertem Wasser gespült und mit Luft getrocknet.

Anschließend wird die mit den Flüssigkeiten der Beispiele 1 bis 35 konditionierte Fläche 60 Sekunden lang mit dem Beschichtungsmittel nach Beispiel 36 behandelt. Danach wird die Fläche mit Luft getrocknet.

Die Scherfestigkeit an Zahnbein ist in Tabelle 2 und an Zahnschmelz in Tabelle 3 zusammengefaßt.

Tabelle 2

| Flüssigkeit nach Beispiel Nr. | 1 | 2 | 3 | 4 | 5 | 6 | 7 | 8 | 9 | 10 | 11 | 12 |
|---|---|---|---|---|---|---|---|---|---|---|---|---|
| Scherfestigkeit (MPa) | 6,1±2,0 | 13,8±3,2 | 10,7±4,4 | 6,9±2,2 | 8,2±3,7 | 13,2±4,4 | 21,6 | 20,9 | 29,7 | 19,9 | 18,9 | 15,0 |

Vergleich

| Flüssigkeit nach Beispiel Nr. | 13 | 14 | 15 | 16 | 17 | 18 | 19 | 20 | 21 | 22 | 23 | 24 |
|---|---|---|---|---|---|---|---|---|---|---|---|---|
| Scherfestigkeit (MPa) | 9,9 | 15,5 | 5,6 | 12,3 | 10,6 | 8,8 | 9,5 | 12,7 | 10,9 | 1,9±0,9 | 3,9±1,3 | 13,4±2,9 |

Tabelle 3

| Flüssigkeit nach Beispiel Nr. | 2 | 6 | 7 | 8 | 9 | 10 | 11 |
|---|---|---|---|---|---|---|---|
| Scherfestigkeit (MPa) | 23,7±3,6 | 24,7±4,3 | 13,1 | 14,9 | 17,1 | 20,5 | 24,3 |

Tabelle 2 (Fortsetzung)

| Flüssigkeit nach Beispiel Nr. | 25 | 26 | 27 | 28 | 29 | 30 | 31 | 32 | 33 | 34 | 35 |
|---|---|---|---|---|---|---|---|---|---|---|---|
| Scherfestigkeit (MPa) | 16,8±5,6 | 14,6±6,7 | 14,7±3,4 | 18,9±2,2 | 8,8±2,5 | 10,6±4,6 | 12,3±4,5 | 10,9±2,5 | 12,7±1,4 | 9,5±2,3 | 5,4±3,1 |

Tabelle 3

| Beispiel Nr. | 25 | 27 | 28 | 29 | 30 | 31 |
|---|---|---|---|---|---|---|
| Scherfestigkeit (MPa) | 10,7±0,9 | 9,9±2,4 | 8,4±1,3 | 16,4±2,7 | 13,6±1,8 | 10,5±1,7 |

**Patentansprüche**

**Patentansprüche für folgende Vertragsstaaten : AT, BE, CH, DE, FR, GB, IT, LI, NL, SE**

1. Flüssigkeiten zur Konditionierung von Zahn- oder Knochensubstanzen, enthaltend in wäßriger Lösung

eine Säure mit einem $pK_S$-Wert von kleiner als +5 und
eine amphotere Aminoverbindung mit einem $pK_S$-Wert im Bereich von 3,9 bis 12,5 und einem $pK_B$-Wert im Bereich von 10,5 bis 13,5, in einem solchen Verhältnis zueinander, daß
die Lösung einen pH-Wert im Bereich 0,1 bis 3,5 aufweist.

**2.** Flüssigkeit nach Anspruch 1, dadurch gekennzeichnet, daß die Säure einen $pK_S$-Wert im Bereich von -9 bis +5 aufweist.

**3.** Flüssigkeit nach den Ansprüchen 1 und 2, dadurch gekennzeichnet, daß die amphotere Aminoverbindung einen $pK_S$-Wert im Bereich von 9,0 bis 10,6 und einen $pK_B$-Wert von 11,5 bis 12,5 aufweist.

**4.** Flüssigkeiten nach den Ansprüchen 1 bis 3, dadurch gekennzeichnet, daß die Lösung einen pH-Wert im Bereich von 1,5 bis 2,5 aufweist.

**5.** Verfahren zur Herstellung von Flüssigkeiten zur Konditionierung von Zahn- oder Knochensubstanz gemäß einem der Ansprüche 1-4, dadurch gekennzeichnet, daß man eine wäßrige Lösung einer Säure mit einem $pK_S$-Wert von kleiner als +5 und
eine amphotere Aminoverbindung mit einem $pK_S$-Wert im Bereich von 3,9 bis 12,5 und einem $pK_B$-Wert im Bereich von 10,5 bis 13,5
umsetzt,
wobei ein pH-Wert im Bereich von 0,1 bis 3,5 eingehalten wird.

**6.** Verwendung von Flüssigkeiten gemaß Ansprüchen 1-4 zur Konditionierung von Zahn- oder Knochensubstanz.

**7.** Verwendung nach Anspruch 6, dadurch gekennzeichnet, daß man nach Auftrag der Flüssigkeit zur Konditionierung ein Beschichtungsmittel anwendet und dann eine Versorgung mit Kunststoff vornimmt.

**Patentansprüche für folgenden Vertragsstaat : ES**

**1.** Verfahren zur Herstellung von Flüssigkeiten zur Konditionierung von Zahn- oder Knochensubstanz, enthaltend in wäßriger Lösung eine Säure mit einem $pK_S$-Wert von kleiner als +5 und eine amphotere Aminoverbindung mit einem $pK_S$-Wert im Bereich von 3,9 bis 12,5 und einem $pK_B$-Wert im Bereich von 10,5 bis 13,5, in einem solchen Verhältnis zueinander, daß die Lösung einen pH-Wert im Bereich 0,1 bis 3,5 aufweist, dadurch gekennzeichnet, daß man eine wäßrige Lösung einer Säure mit einem $pK_S$-Wert von kleiner als +5 und eine amphotere Aminoverbindung mit einem $pK_S$-Wert im Bereich von 3,9 bis 12,5 und einem $pK_B$-Wert im Bereich von 10,5 bis 13,5 umsetzt, wobei ein pH-Wert im Bereich von 0,1 bis 3,5 eingehalten wird.

**2.** Verfahren nach Anspruch 1, dadurch gekennzeichnet, daß die Säure einen $pK_S$-Wert im Bereich von -9 bis +5 aufweist.

**3.** Verfahren nach den Ansprüchen 1 und 2, dadurch gekennzeichnet, daß die amphotere Aminoverbindung einen $pK_S$-Wert im Bereich von 9,0 bis 10,6 und einen $pK_B$-Wert von 11,5 bis 12,5 aufweist.

**4.** Verfahren nach den Ansprüchen 1 bis 3, dadurch gekennzeichnet, daß die Lösung einen pH-Wert im Bereich von 1,5 bis 2,5 aufweist.

**Claims**

**Claims for the following Contracting States : AT, BE, CH, DE, FR, GB, IT, LI NL, SE**

**1.** Liquids for conditioning tooth or bone matter containing, in aqueous solution, an acid with a $pK_A$ value of less than +5 and an amphoteric amino compound with a $pK_A$ value in the range from 3.9 to 12.5 and a $pK_B$ value in the range from 10.5 to 13.5, in such a ratio to one another that the solution has a pH in the range from 0.1 to 3.5.

**2.** Liquid according to Claim 1, characterised in that the acid has a $pK_A$ value in the range from -9 to +5.

3. Liquid according to Claims 1 and 2, characterised in that the amphoteric amino compound has a $pK_A$ value in the range from 9.0 to 10.6 and a $pK_B$ value of 11.5 to 12.5.

4. Liquids according to Claims 1 to 3, characterised in that the solution has a pH in the range from 1.5 to 2.5.

5. Process for the preparation of liquids for conditioning tooth or bone matter according to one of Claims 1 - 4, characterised in that an aqueous solution of an acid with a $pK_A$ value of less than +5 and an amphoteric amino compound with a $Pk_A$ value in the range from 3.9 to 12.5 and a $pK_B$ value in the range from 10.5 to 13.5 are reacted, a pH in the range from 0.1 to 3.5 being maintained.

6. Use of liquids according to Claims 1 - 4 for conditioning tooth or bone matter.

7. Use according to Claim 6, characterised in that, after application of the liquid for conditioning, a coating agent is used and then treatment with synthetic material is carried out.

**Claims for the following Contracting State : ES**

1. Process for the preparation of liquids for conditioning tooth or bone matter containing, in aqueous solution, an acid with a $pK_A$ value of less than +5 and an amphoteric amino compound with a $pK_A$ value in the range from 3.9 to 12.5 and a $pK_B$ value in the range from 10.5 to 13.5, in such a ratio to one another that the solution has a pH in the range from 0.1 to 3.5, characterised in that an aqueous solution of an acid with a $pK_A$ value of less than +5 and an amphoteric amino compound with a $pK_A$ value in the range from 10.5 to 13.5 are reacted, a pH in the range from 0.1 to 3.5 being maintained.

2. Process according to Claim 1, characterised in that the acid has a $pK_A$ value in the range from -9 to +5.

3. Process according to Claims 1 and 2, characterised in that the amphoteric amino compound has a $pK_A$ value in the range from 9.0 to 10.6 and a $pK_B$ value of 11.5 to 12.5.

4. Process according to Claims 1 to 3, characterised in that the solution has a pH in the range from 1.5 to 2.5.

**Revendications**
**Revendications pour les Etats contractants suivants : AT, BE, CH, DE, FR, GB, IT, LI, NL, SE**

1. Liquides pour le traitement prélable des dents ou des os, contenant en solution aqueuse,
   un acide ayant une valeur de pKa inférieure à +5 et
   un composé aminé amphotère ayant une valeur de pKa dans l'intervalle de 3,9 à 12,5 et une valeur de pKb dans l'intervalle de 10,5 à 13,5, dans des proportions relatives telles que la solution présente un pH dans l'intervalle de 0,1 à 3,5.

2. Liquide selon la revendications 1, caractérisé en ce que l'acide a une valeur de pKa dans l'intervalle de -9 à +5.

3. Liquide selon les revendications 1 et 2, caractérisé en ce que le composé aminé amphotère a une valeur de pKa dans l'intervalle de 9,0 à 10,6 et une valeur de pKb de 11,5 à 12,5.

4. Liquides selon les revendications 1 à 3, caractérisés en ce que la solution a un pH dans l'intervalle de 1,5 à 2,5.

5. Procédé de préparation de liquides pour le traitement préalable des dents ou des os selon une des revendications 1 à 4, caractérisé en ce que l'on fait réagir une solution aqueuse d'un acide ayant une valeur de pKa inférieure à +5 et
   un composé aminé amphotère ayant une valeur de pKa dans l'intervalle de 3,9 à 12,5 et une valeur de pKb dans l'intervalle de 10,5 à 13,5 en maintenant un pH dans l'intervalle de 0,1 à 3,5.

6. Utilisation des liquides selon les revendications 1 à 4 pour le traitement préalable des dents ou des os.

7. Utilisation selon la revendication 6, caractérisée en ce que, après application du liquide de traitement préalable, on utilise un produit de revêtement puis on procède à une restauration à l'aide d'une résine synthétique.

**Revendications pour l'Etat Contractant suivant : ES**

1. Procédé de préparation de liquides pour le traitement préalable des dents ou des os, contenant en solution aqueuse un acide ayant une valeur de pKa inférieure à +5 et un composé aminé amphotère présentant une valeur de pKa dans l'intervalle de 3,9 à 12,5 et une valeur de pKb dans l'intervalle de 10,5 à 13,5, dans des proportions relatives telles que la solution présente un pH dans l'intervalle de 0,1 à 3,5, caractérisé en ce que l'on fait réagir une solution aqueuse d'un acide ayant une valeur de pKa inférieure à +5 et un composé aminé amphotère ayant une valeur de pKa dans l'intervalle de 3,9 à 12,5 et une valeur de pKb dans l'intervalle de 10,5 à 13,5 en maintenant un pH dans l'intervalle de 0,1 à 3,5.

2. Procédé selon la revendication 1, caractérisé en ce que l'acide a une valeur de pKa dans l'intervalle de -9 à +5.

3. Procédé selon les revendications 1 et 2, caractérisé en ce que le composé aminé amphotère a une valeur de pKa dans l'intervalle de 9,0 à 10,6 et un pKb de 11,5 a 12,5.

4. Procédé selon les revendications 1 à 3, caractérisé en ce que la solution a un pH dans l'intervalle de 1,5 a 2,5.